# EUROPEAN PATENT APPLICATION

(11) **EP 0 823 766 A1**
(43) Date of publication of application: **11.02.1998**
(21) Application number: 97113503.3
(22) Date of filing: 05.08.1997
(51) Int. Cl.: H02J 7/02

(54) **Cooling charge cable for electric vehicle**

(30) Priority: 07.08.1996 JP 208601/96; 09.10.1996 JP 268863/96
(71) Applicant: Sumitomo Wiring Systems, Ltd., Yokkaichi-shi Mie-ken (JP); SUMITOMO ELECTRIC INDUSTRIES, LTD., Osaka-shi, Osaka 541 (JP)
(72) Inventor: Watanabe, Kunihiko, Yokkaichi-shi, Mie (JP); Yoshimura, Masanobu, Yokkaichi-shi, Mie (JP); Kuku, Heiji, Yokkaichi-shi, Mie (JP); Kanagawa, Shuichi, Yokkaichi-shi, Mie (JP); Arisaka, Shuji, Konohana-ku, Osaka-shi, Osaka (JP); Shimada, Toshiro, Konohana-ku, Osaka-shi, Osaka (JP); Deguchi, Hiroshige, Konohana-ku, Osaka-shi, Osaka (JP)
(74) Representative: KUHNEN, WACKER & PARTNER

(57) **Abstract**

An electric vehicle charging cooling cable disposed between a charging coupler for induction charging set in an electric vehicle and an external charging power supply, comprises: a plurality of conductive wires in a sheath; and a pair of flexible refrigerant pipes disposed in the sheath together with the conductive wires. In the flexible refrigerant pipes, a refrigerant is circulated to cool the charging coupler.

## Description

### BACKGROUND OF THE INVENTION

This invention relates to a charging cable using electromagnetic induction for charging an electric vehicle.

A connector using electromagnetic induction for charging a battery in an electric vehicle has various advantages because it can supply electric power to the electric vehicle in non-contact not via electric contacts; various structures of the connectors are designed. The basic structure is provided by applying the principles of a transformer wherein coils are wound around primary and secondary cores and when an electric vehicle is charged, an alternating current is caused to flow into the primary coil for causing the secondary coil to produce an electromotive force by electromagnetic coupling, as disclosed in Unexamined Japanese Patent Publications Nos. 5-258962, 5-260671, 6-14470, or the like.

However, in such a charging system, the cores and coils generate heat at the charging time and the heat needs to be removed using a refrigerant such as cooling water to made be a large capacity.

However, if a conduit line for causing a refrigerant to flow is provided aside from a power supply cable, the number of cables is increased and the cables become hard to handle and a foot becomes easily caught on the cables accidentally; the refrigerant conduit line is scratched and an accident such as a refrigerant leak easily occurs.

### SUMMARY OF THE INVENTION

It is therefore an object of the invention to provide an electric vehicle charging cooling cable which is easy to handle and can prevent damage to refrigerant pipes, etc., although it enables forced cooling using a refrigerant.

To the end, according to the invention, there is provided an electric vehicle charging cooling cable comprising conductive wires in a sheath and being disposed between a charging coupler for induction charging set in an electric vehicle and an external charging power supply, characterized by flexible refrigerant pipes being disposed in the sheath together with the conductive wires for circulating a refrigerant in the charging coupler.

With the above-mentioned structure, the cable contains the refrigerant pipes for circulating refrigerants as well as the conductive wires, so that the electric vehicle can be charged and the coils, cores, etc., can be cooled by means of one charging cable. Handling is easy as compared with a case where the refrigerant pipes are provided aside from the charging cable, and the refrigerant pipes are housed in the sheath, so that damage to the refrigerant pipes caused by catching, friction, etc., can be prevented.

Further, according to the invention, the refrigerant pipes are placed spirally in the sheath. According to this structure, because the refrigerant pipes are placed spirally in the cable, thus resistance of the refrigerant pipes to bending the cable is lessened and the cable is easily bent. That is, the cable is easy to handle when the electric vehicle is charged or the cable is stored.

Furthermore, the conductive wires are placed in a common sheath and consist of an inner core comprising a plurality of litz wires twisted each comprising a plurality of insulation strands bundled and an outer core comprising a plurality of litz wires and being placed coaxially with the inner core so as to surround the outer periphery of an interlayer insulating layer disposed on the outer periphery of the inner core.

According to this structure, each core consists of the litz wires, so that a resistance increase caused by the skin effect when a high frequency current is caused to flow lessens and an energization loss lessens. Moreover, the outer core is placed surrounding the inner core concentrically. Thus, if a shield layer is not provided, magnetic fields produced by currents flowing into the cores negate each other. Resultantly, a magnetic flux is not leaked to the outside and can be prevented from becoming a noise source.

Furthermore, the refrigerant are disposed in the inner core. If a coaxial structure is adopted, heat accumulates in the inner core more easily than in the outer core. However, the refrigerant pipes are disposed in the inner core, so that a heat exchange is made easily between the inner core and a refrigerant and a temperature rise in the cable can be prevented reliably.

Moreover, according to the invention, the litz wires making up the outer core are placed spirally on the outer periphery of the interlayer insulating layer and that the refrigerant pipes are placed spirally in parallel with the litz wires making up the outer core.

According to this structure, the refrigerant pipes are placed spirally in parallel with the litz wires making up the outer core in the cable. Thus, as compared with a case where the refrigerant pipes are placed linearly, bending the cable is facilitated, enhancing easy handling when the cable is used or stored.

In addition, when the outer core is prepared, the cable is prepared while the refrigerant pipes are placed simultaneously with the litz wires, thus manufacturing of the cable is facilitated.

Still further, the refrigerant pipes are connected to a cooler provided for the external charging power supply and heat of refrigerants circulated through the refrigerant pipes is radiated by the cooler.

According to this structure, refrigerants are circulated while they are cooled by the cooler, thus waste of the refrigerants is eliminated, reducing the running cost.

### BRIEF DESCRIPTION OF THE DRAWINGS

Figure 1 is a side view to show how an electric vehicle is charged;
Figure 2 is a longitudinal sectional view of a charging coupler and a receptacle of an electric vehicle according to a first embodiment of the invention;
Figure 3 is an enlarged side view to show a connection part of an electrically conductive pipe of the first embodiment of the invention;
Figure 4 is a partially cutaway view in perspective of a charging cable of the first embodiment of the invention;
Figure 5 is a sectional view of the charging cable of the first embodiment of the invention;
Figure 6 is a partially cutaway view in perspective of a charging cable according to a second embodiment of the invention;
Figure 7 is a sectional view of the charging cable of the second embodiment of the invention;
Figure 8 is a sectional view of a charging cable of a third embodiment of the invention;
Figure 9 is a sectional view of a charging cable of the other embodiment of the invention;
Figure 10 is a sectional view of a charging cable of the other embodiment of the invention; and
Figure 11 is a cutaway view in perspective of a charging cable of the other embodiment of the invention.

### DETAILED DESCRIPTION OF THE PREFERRED EMBODIMENTS

### First Embodiment

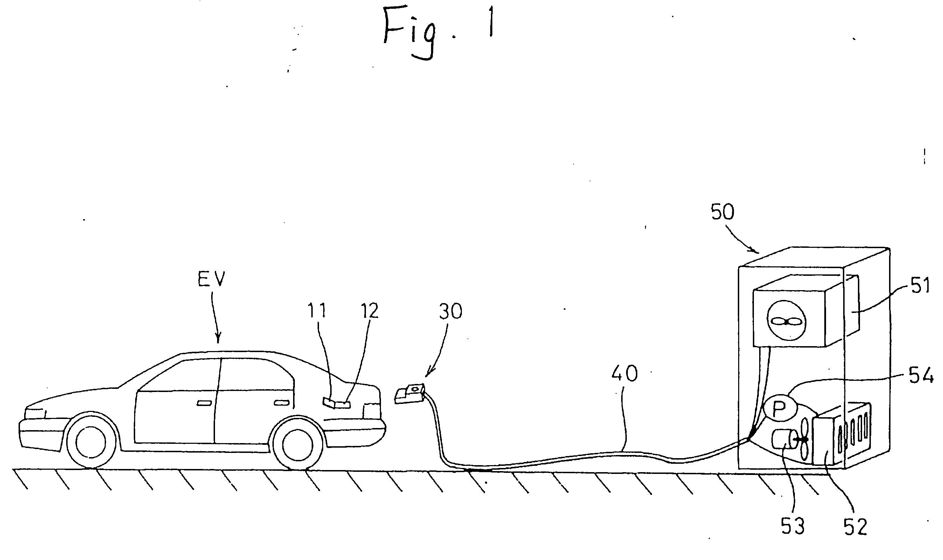

A first embodiment of the invention will be discussed with reference to Figures 1 to 5.

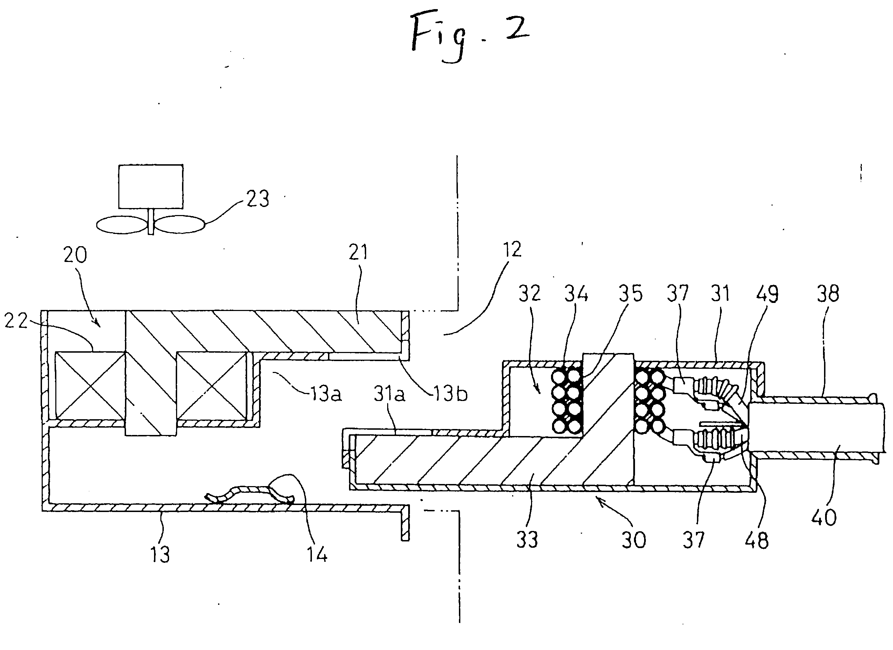

Before a charging cable 40 of the invention is described, how an electric vehicle EV is charged will be discussed with Figures 1 and 2. A receptacle 12 that can be opened and closed with a lid 11, for example, is formed on the outside of the body of the electric vehicle EV, and a charging coupler 30 (described later) can be inserted into the receptacle. The charging cable 40 of the invention is connected to the charging coupler 30 for connection to a charging system 50. The charging system 50 comprises a high frequency power supply 51 equivalent to an external charging power supply for outputting a 100-kHz high frequency voltage, for example, an air-cooling cooler 52 for radiating heat from cooling water as a refrigerant (described later), and an air cooling fan 53.

A coupler reception case 13 forming a recess 13a opened outward is attached to the receptacle 12 of the electric vehicle EV and a secondary coil unit 20 is placed in the coupler reception case 13. The secondary coil unit 20 comprises a secondary coil 22 wound around a second core 21 made of ferrite, for example, and is cooled by a cooling fan provided in the body. An output terminal of the secondary coil 22 is connected to a charging circuit for charging a power battery (not shown) of a power storage unit of the electric vehicle EV; a high frequency electromotive force induced to the secondary coil 22 is rectified and the power battery can be charged.

The secondary core 21 is formed like a shape of bending a square pole as an L letter and is fixed to the coupler reception case 13 with the long side of the L letter of the secondary core set on its side. The short side of the L letter extends downward and its lower end part penetrates the coupler reception case 13 and slightly projects into the recess 13a. The tip of the long side of the L letter is exposed to the recess 13a through an opening 13b made in the front end of the coupler reception case 13. A plate spring 14 is attached to the bottom of the recess 13a of the coupler reception case 13 for energizing the charging coupler 30 inserted into the recess 13a upward (the side of the secondary coil unit 20).

On the other hand, the charging coupler 30 comprises a primary coil 32 and a primary core 33 housed in a housing 31. The same core as the secondary core 21 is used as the primary core 33. The primary core 33 is fixed to the housing 31 with the long side of the L letter of the primary core 33 along the inside of the housing 31. The short side of the L letter of the primary core 33 extends upward on the base of the housing 31 and the top end face of the short side penetrates the housing 31 and projects to the outside. The tip of the long side of the L letter is exposed to the top face through an opening 31a made in the front end of the housing 31. Therefore, if the charging coupler 30 is inserted into the recess 13a of the coupler reception case 13 of the electric vehicle EV, the top face of the tip of the long side of the primary core 33 is opposed to the lower end face of the short side of the secondary core 21 and the top face of the short side of the primary core 33 is opposed to the lower face of the tip of the long side of the secondary core 21. The plate spring 14 placed on the bottom face of the recess 13a of the coupler reception case 13 energizes the charging coupler 30 upward, whereby the opposed faces of both the cores 21 and 33 almost come into contact with each other and both the cores 21 and 33 form a magnetic circuit of a single closed loop.

The primary coil 32 is formed on the short side of the primary core 33 by winding an electrically conductive pipe 34 more than one turn. In the embodiment, the electrically conductive pipe 34, which is made of a copper alloy, is 5 mm in diameter, 0.5 mm in wall thickness, and about 25 mm in inner roll diameter, for example. Heat-transferable silicone grease, etc., for example, is applied to the inner periphery of the primary coil 32 between the primary coil 32 and the primary core 33, whereby heat transferability is enhanced between the primary coil 32 and the primary core 33 as compared with a state in which the silicone grease is not applied, and the primary coil 32 is wound around the primary core 33 so that heat can be transferred. Although not shown, the electrically conductive pipe 34 is covered with enamel on both inner and outer surfaces for insulation.

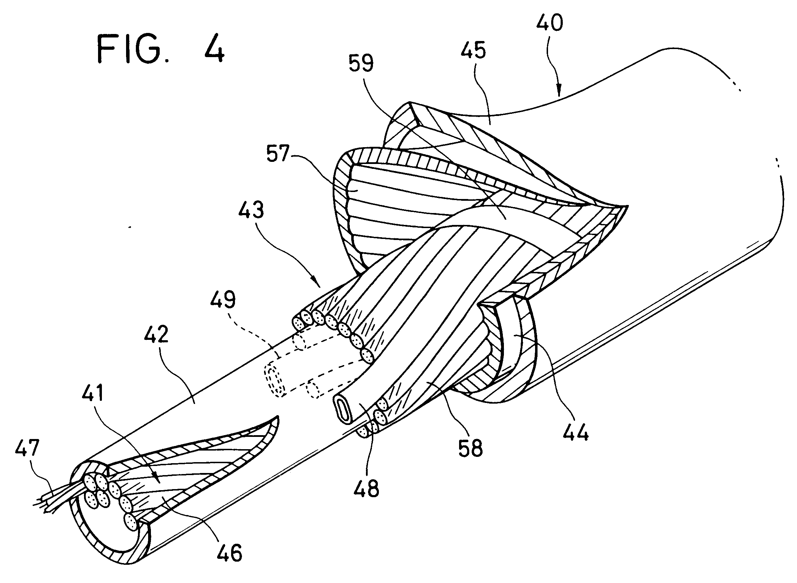

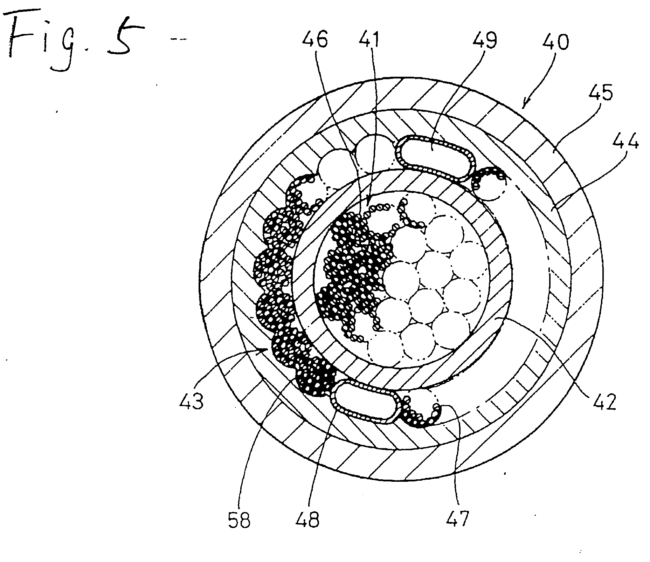

Figures 4 and 5 show the charging cable 40 of the first embodiment of the invention. The charging cable 40 has a two-core structure wherein an interlayer insulating layer 42 made of polyvinyl chloride (PVC) is provided surrounding the outer periphery of an inner core 41 and an outer core 43 is placed so as to surround the outer periphery of the interlayer insulating layer 42; an outer insulating layer 44 made of PVC is placed on the outside of the outer core 43 and a sheath 45 made of PVC is located on the outside of the outer insulating layer 44.

The inner core 41 comprises a bundle of 19 litz wires 46 twisted (in Figure 4 or 5, some of the litz wires are omitted for convenience). The litz wire 46 is of a general structure of bundling a plurality of insulation strands 47. The type of insulation strand 47 and the number of insulation strands 47 are determined matching the required energization capacity, frequency, etc. In the embodiment, polyurethane insulation copper lines 0.18 mm in diameter are bundled and twisted, for example.

On the other hand, the outer core 43 is made up of litz wires 58 similar to the litz wires 46 making up the inner core 41 and is spirally parallel placed with the 19 litz wires 58 surrounding the outer periphery of the interlayer insulating layer 42. Two refrigerant pipes 48 and 49 forming go and return passages at positions facing each other almost on the circumference are provided in parallel with the spiral of the litz wires 58 between the litz wires 58 making up the outer core 43. The refrigerant pipes 48 and 49 are manufactured of a synthetic resin having heat resistance and flexibility, such as a silicon resin, and is substantially oval in cross section.

Tying tape 59 is wound spirally in opposite orientation to that of the litz wires 58 and the refrigerant pipes 48 and 49 so as to cover the outer periphery of the litz wires 58 of the outer core 43. The outer insulating layer 44 is formed on the inner periphery with a holding groove 57 along the spiral placement of the litz wires 58 and the refrigerant pipes 48 and 49 and the litz wires 58 and the refrigerant pipes 48 and 49 are fitted into the holding groove 57.

The cores 41 and 43 of the charging cable 40 are connected to an output terminal of the high frequency power supply 51 in the charging system 50. The refrigerant pipe 48 as the go passage is coupled to a circulating pump 54 in the charging system and the refrigerant pipe 49 as the return passage is coupled to the cooler 52.

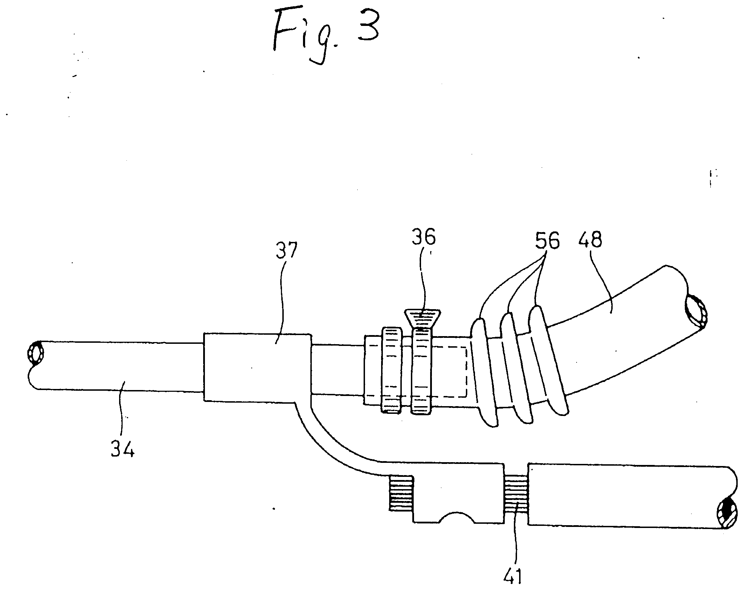

Figure 3 shows a connection structure of the primary coil 32 and the charging cable 40. That is, the refrigerant pipe 48 is fitted into the end of the electrically conductive pipe 34 and is coupled to the electrically conductive pipe 34 in water-sealing relation at a pipe clamp 36. The refrigerant pipe 48 is formed at the coupling end with a plurality of water drip sheds 56 integrally. If water drops flow from the electrically conductive pipe 34 due to dew condensation to the electrically conductive pipe 34 or from the refrigerant pipe 48, the water drip sheds 56 can shut off the water drops flowing into the opposite side. The refrigerant pipe 48 of the go passage is coupled to the end of the electrically conductive pipe 34 on the inner periphery of the primary coil 32 and the refrigerant pipe 49 of the return passage is coupled to the end on the outer periphery of the primary coil 32, so that cooling water flows from the inner periphery of the primary coil 32 to the outer periphery thereof.

An energization terminal 37 is connected to a part near the coupling part of the electrically conductive pipe 34 to the refrigerant pipe 48 by brazing, for example, and the inner core 41 of the charging cable 40 is crimped at the energization terminal 37. In the charging coupler 30, the charging cable 40 penetrates a tube 38 also used as a handle integrally projecting to the base of the housing 31.

The embodiment has the structure described above and the function of the embodiment will be discussed. To charge the electric vehicle EV, first the charging coupler 30 is inserted into the receptacle 12 of the car body. Then, it is inserted into the depth of the reception case 13 and is pushed against the secondary coil unit 20 by the plate spring 13 in the reception case 13, placing both the cores 21 and 33 in junction, thereby forming a magnetic circuit of a closed loop. When a power switch of the charging system (not shown) is turned on, the circulating pump 54 and the cooling fan 53 are started and the high frequency power supply 51 operates, applying a high frequency voltage to the primary coil 32 through the charging cable 40. As the primary coil 32 is excited, an electromotive force occurs on the secondary coil 22, based on which the power battery of the electric vehicle EV is charged.

As high frequency current flows into the primary coil 32, the electrically conductive pipe 34 itself forming the primary coil 32 and the primary core 33 generate heat. However, the circulating pump 54 is run as described above, so that cooling water flows through the electrically conductive pipe 34 via the refrigerant pipe 48 of the go passage of the charging cable 40 and is returned from the cooler 52 to the circulating pump 54 via the refrigerant pipe 49 of the return passage of the charging cable 40, producing a circulation flow of the refrigerant. Thus, heat generated at the electrically conductive pipe 34 is immediately transferred to the cooling water flowing through the electrically conductive pipe 34, carried to the cooler 52, and cooled by the air cooling fan 53, then circulated. Therefore, the electrically conductive pipe 34 is immediately cooled if a large amount of Joule heat is generated during the charging, and the temperature of the primary coil 32 can be reliably prevented from largely rising. Heat generated at the primary core 33 due to a hysteresis loss or an eddy current is transferred to the electrically conductive pipe 34 forming the inner periphery of the primary core 32, passes through the refrigerant pipe 49 of the return passage of the charging cable 40, and is carried to the cooler 52 by the cooling water flowing through the electrically conductive pipe 34. Therefore, heat generation of the primary core 33 can be prevented reliably.

Heat generated in the secondary coil unit 20 is cooled by the air cooling fan 23 in the car body.

Thus, the embodiment produces the following effects:

Since the refrigerant pipes 48 and 49 are integrated with the charging cable 40 so that they are wound around the outer periphery of the interlayer insulating layer 42, the electric vehicle EV can be charged and the coils, etc., can be cooled by means of one charging cable 40. Handling is easy as compared with a case where the refrigerant pipes are provided aside from the charging cable, and the refrigerant pipes 48 and 49 are housed in the inside of the sheath 45, so that damage to the refrigerant pipes caused by catching, friction, etc., can be prevented.

If the coil 34, the core 34, the charging cable 40, and the like generate heat when a high frequency current is caused to flow into the inner core 41 and the outer core 43, a refrigerant is caused to flow into the refrigerant pipes 48 and 49 at the same time, thus the heated components can be cooled.

Since the refrigerant pipes 48 and 49 serve as the go and return passages of a refrigerant, the refrigerant can be circulated. Since the circulating pump 54 is provided at one end of the charging cable 40, the refrigerant can be circulated on the whole charging cable 40. Cooling water is circulated while it is cooled by the cooler 52, thus waste of the refrigerant is eliminated, reducing the running cost. Since the charging system 50 contains the cooler 52, the entire charging facilities become compact.

Moreover, the two refrigerant pipes 48 and 49 are placed spirally in parallel with the litz wires 58 making up the outer core 43 in the charging cable 40, so that the charging cable is easily bent, enhancing easy handling when the charging cable 40 is used or stored. In addition, when the outer core 43 is prepared, the charging cable 40 is prepared while the refrigerant pipes 48 and 49 are placed simultaneously with the litz wires 58, thus manufacturing of the charging cable 40 is facilitated.

Since the two cores 41 and 43 are placed in the charging cable 40, a current can be caused to flow through the cores 41 and 43 and only one charging cable 40 is needed to distribute power to the electric vehicle EV. The core 41, 43 consists of the litz wires 46, 58, so that a resistance increase caused by the skin effect when a high frequency current is caused to flow lessens and an energization loss lessens. Moreover, the outer core 43 is placed surrounding the inner core 41 concentrically. Thus, if a shield layer is not provided, magnetic fields produced by currents flowing into the cores 41 and 43 negate each other. Resultantly, a magnetic flux is not leaked to the outside and can be prevented from becoming a noise source.

Since both the cores 41 and 43 are placed concentrically, the space efficiency is excellent and the whole diameter dimension can be lessened. In this connection, if the same number of the same litz wires 46, 58 as those in the embodiment are bundled to parallel two-core electric lines, the outer diameter of the cable becomes about 20 mm. In the concentric placement in the embodiment, the outer diameter can be set to about 18 mm and both diameter narrowing and weight saving can be accomplished in the same energization capacity.

In the embodiment, the litz wires 58 making up the outer core 43 are placed spirally. Thus, if an electric distribution varies from one litz wire 58 to another, the variations can be evened as the whole charging cable 40, so that a leak of a magnetic flux can be prevented reliably.

### Second Embodiment

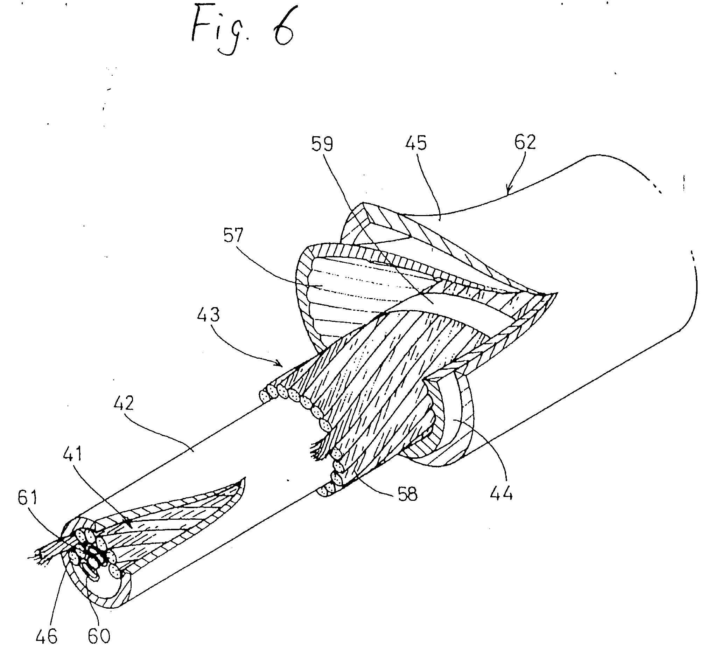

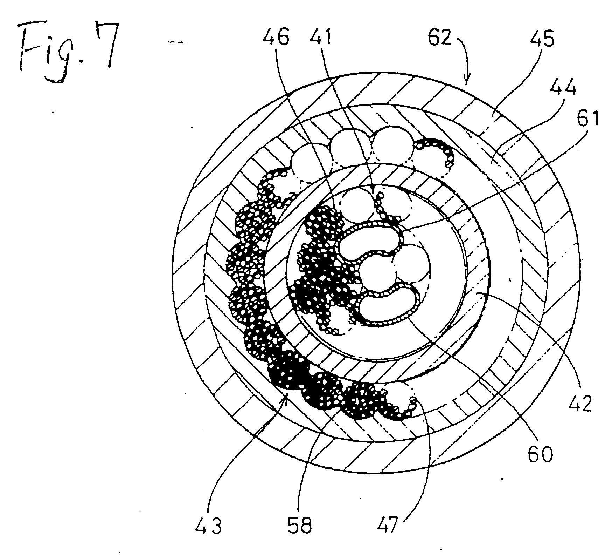

Next, a second embodiment of the invention will be discussed with reference to Figures 6 and 7.

Parts identical with or similar to those previously described with reference to Figures 4 and 5 are denoted by the same reference numerals in Figures 6 and 7 and will not be discussed again.

The first and second embodiments differ in positions of refrigerant flow passages. In the second embodiment, two refrigerant pipes 60 and 61 disposed in a charging cable 62 are placed in parallel with an inner core 41.

Such a structure would be able to produce similar functions and effects to those of the first embodiment.

Further, inner and outer cores 41 and 43 of the charging cable 62 are almost equal in heat output of Joule heat, but heat accumulates more easily in the inner core 41. In view of this point, the two refrigerant pipes 60 and 61 are provided in the inner core 41 in the embodiment, so that a heat exchange is made easily between the inner core 41 and a refrigerant and a temperature rise in the charging cable 62 can be prevented reliably.

### Third Embodiment

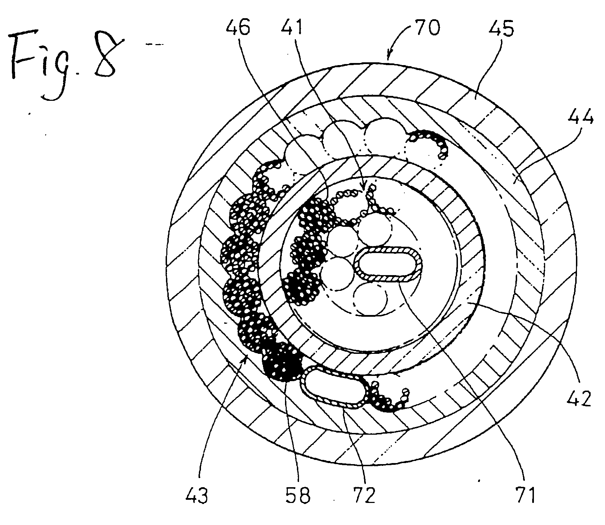

Next, a third embodiment of the invention will be discussed with reference to Figure 8.

Parts identical with or similar to those previously described with reference to Figure 5 are denoted by the same reference numerals in Figure 8 and will not be discussed again.

The first and third embodiments differ in positions of refrigerant flow passages. In the third embodiment, of two refrigerant pipes 71 and 72 disposed in a charging cable 70, the refrigerant pipe 71 as a go passage is placed at the center of an inner core 41 and the refrigerant pipe 72 as a return passage is placed in parallel with an outer core 43.

Such a structure would be able to produce similar functions and effects to those of the first embodiment.

As described in the second embodiment, heat accumulates in the inner core 41 more easily than in the outer core 43. Thus, the refrigerant pipe 71 of the go passage through which a low-temperature refrigerant flows is placed along the inner core 41 and the refrigerant pipe 72 of the return passage through which a higher-temperature refrigerant than that refrigerant flows is placed along the outer core 43 having a good heat radiation property. Resultantly, the inner core 41 can be cooled efficiently and a temperature rise in the charging cable 70 can be prevented more reliably.

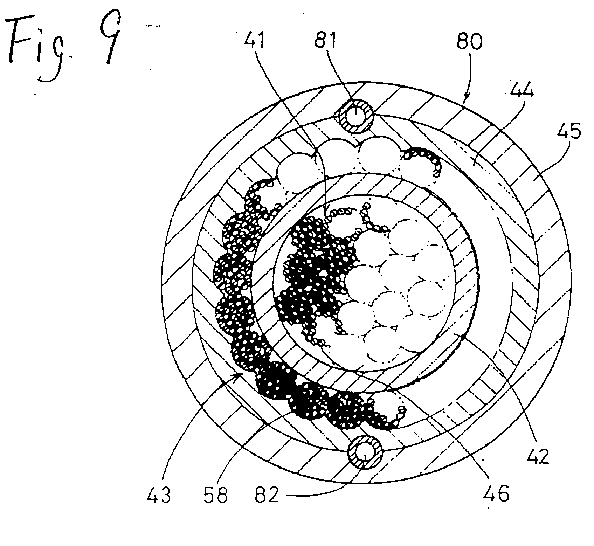

Figures 9-11 show miscellaneous embodiments of the invention.

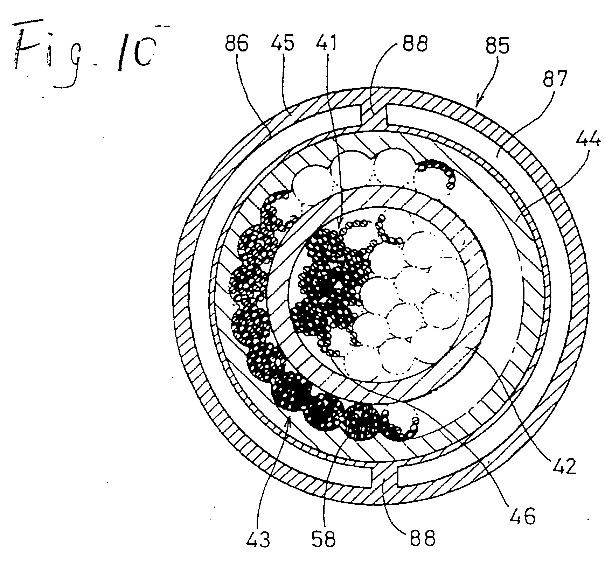

Parts identical with or similar to those previously described with reference to Figure 5 are denoted by the same reference numerals in Figures 9 and 10 and will not be discussed again.
(1) In Figure 9, two refrigerant pipes 81 and 82 of a charging cable 80 are placed in line between an outer insulating layer 44 and a sheath 45. Such a structure produces the effect of facilitating preparation of the charging cable 80.
   The refrigerant pipes 81 and 82 can also be placed spirally in the sheath 45. In doing so, the charging cable 80 can be easily bent.
(2) In Figure 10, the outer layer sides of two refrigerant pipes 86 and 87 of a charging cable 85 are formed like a jacket also serving as a sheath 45, and partitions 88 are disposed between the two refrigerant pipes 86 and 87 for preventing refrigerants on go and return passages from being mixed. Such a structure would be able to increase the amounts of refrigerants flowing through the refrigerants 86 and 87, so that the cooling effect can be enhanced.
   In the embodiment, the refrigerant pipes 86 and 87 may be formed like a jacket also serving as an outer insulating layer 44 and a sheath 45 may be disposed on the outer periphery.

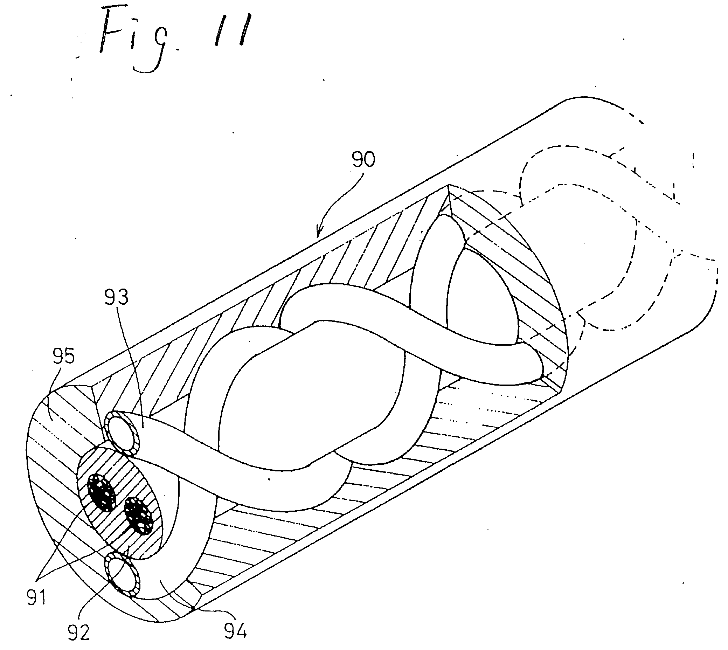

(3) In Figure 11, a charging cable 90 has two cores 91, two refrigerant pipes 93 and 94 forming go and return passages are wound around the outer peripheries of the cores 91 spirally in opposite directions via an insulating layer 92, and a sheath 95 is formed on the outer periphery for integrating the whole. The refrigerant pipes 93 and 94 are manufactured of a soft synthetic resin resistant to heat, such as a silicon resin.

The invention is not limited to the embodiments; for example, the following are contained in the technical scope of the invention:
i) The number of refrigerant pipes is not limited to two and any number of pipes may be provided. Only the go or return passage may be formed like a jacket as shown in Figure 10.
ii) The refrigerant may be placed in the charging cable linearly rather than spirally.
iii) Water is not necessarily used as refrigerants circulated in the refrigerant pipes and hydrocarbon family solvents such as oil and chlorofluorocarbon may be used. If a solvent having a low boiling point is used as a refrigerant, it can be circulated as liquid on the refrigerant pipe of the go passage and as gas on the refrigerant pipe of the return passage and can be circulated while it is restored from the gas to the liquid by the cooler. Such a structure would be able to enhance the cooling effect.
iv) If the refrigerant pipes are coupled to the secondary coil unit in water-sealing relation, the coil unit of the automobile can also be charged while it is cooled. At the time, four refrigerant pipes may be provided for the primary and secondary coils.
v) One conductive wire and one refrigerant pipe are placed in a cable and two cables of such a structure may be used to charge the electric vehicle.

## Claims

1. An electric vehicle charging cooling cable disposed between a charging coupler for induction charging set in an electric vehicle and an external charging power supply, said cable comprising:
conductive wires in a sheath; and
a pair of flexible refrigerant pipes disposed in said sheath together with said conductive wires for circulating a refrigerant in the charging coupler.

2. The electric vehicle charging cooling cable as claimed in claim 1, wherein at least one of said refrigerant pipes is placed spirally in said sheath.

3. The electric vehicle charging cooling cable as claimed in claim 1, wherein said conductive wires are placed in a common sheath and consist of:
an inner core including a plurality of litz wires twisted each having a plurality of insulation strands bundled;
an interlayer insulating layer disposed on an outer periphery of said inner core; and
an outer core including a plurality of litz wires and being placed coaxially with said inner core so as to surround an outer periphery of said interlayer insulating layer.

4. The electric vehicle charging cooling cable as claimed in claim 3, wherein at least one of said refrigerant pipes is disposed in said inner core.

5. The electric vehicle charging cooling cable as claimed in claim 3, wherein the litz wires of said outer core are placed spirally on the outer periphery of the interlayer insulating layer and at least one of said refrigerant pipe is placed spirally in parallel with the litz wires said outer core.

6. The electric vehicle charging cooling cable as claimed in claim 3, wherein the litz wires of said outer core are placed spirally on the outer periphery of the interlayer insulating layer and one of said refrigerant pipe is placed spirally in parallel with the litz wires said outer core while the other of said refrigerant pipes is disposed in said inner core.

7. The vehicle charging cooling cable as claimed in claim 1, wherein said refrigerant pipes are connected to a cooler provided for the external charging power supply and heat of refrigerants circulated through said refrigerant pipes is radiated by the cooler.
